# DEMANDE DE BREVET EUROPEEN

(11) **EP 4 368 613 A1**
(43) Date de publication de la demande: **15.05.2024**
(21) Numéro de dépôt: 22205955.2
(22) Date de dépôt: 08.11.2022
(51) Int. Cl.: C07D 237/30, C07D 403/12, C12Q 1/00, G01N 33/50

(54) **DÉRIVÉS DE DICYANOMETHANIDES DE PHTHALAZINIUM**

(71) Demandeur: aeChem Life Technologies Sàrl, 1870 Monthey (CH)
(72) Inventeur: Condemi, Enrico Antonino, 1010 Lausanne (CH)
(74) Mandataire: P&TS SA (AG, Ltd.)

(57) **Abrégé**

La présente invention propose de nouveaux dérivés de dicyanomethanides de phthalazinium (I), un procédé de fabrication et leur utilisation dans la fonctionnalisation de peptides et de protéines, dans la fixation de polymères biologiques sur un support solide à des fins de diagnostic et dans l'élaboration de nouveaux tests diagnostiques.

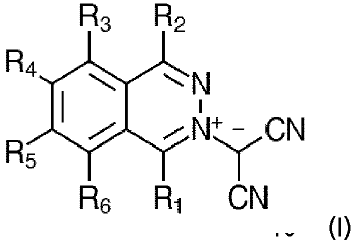

## Description

### Domaine technique

La présente invention concerne les dérivés de dicyanomethanides de phthalazinium et leur utilisation à des fins médicales et/ou de diagnostic.

### Etat de la technique

Dans le domaine médical, une étape de diagnostique est souvent nécessaire. Des tests simples sur supports solides permettent d'obtenir un résultat rapide, au moins préliminaire, sans devoir faire d'analyses complètes en laboratoire. Ces tests sont en général de type immunitaires, ou nécessitent un appariement de molécules spécifiques, et comportent un indicateur visuel tel qu'une couleur. D'autres méthodes de détection, comme la scintigraphie sont parfois utilisées. Dans tous les cas, au moins une partie des composants nécessaires au diagnostique doit être greffée de manière permanente sur un support solide, qu'il soit en verre, en matériau synthétique ou d'une autre nature. Cela implique l'utilisation d'un lien et d'une réaction de couplage avec un biosenseur, lequel peut être un peptide, un fragment d'ADN ou d'ARN ou toute autre biomolécule d'intérêt. Le lien doit en outre être greffé sur le support solide. De telles opérations nécessitent le plus souvent de modifier chimiquement le biosenseur à greffer sur le support de sorte à ce qu'il réagisse avec le lien, au risque d'en modifier ses propriétés ou de le dénaturer. Par ailleurs, les liens utilisés sont souvent développés spécifiquement pour un diagnostique donné, ce qui requiert des efforts de développement pouvant retarder le développement de tels dispositifs de diagnostiques.

De nombreuses méthodes de greffage d'acides nucléiques sur un support solide, à des fins de test diagnostique par exemple, impliquent l'utilisation de molécules organiques, telles que celles décrites dans EP1075544.

Dans le domaine des diagnostiques sur support solide, l'étape de détection nécessite le plus souvent une réaction chimique. C'est le cas notamment des tests de type ELISA, où une enzyme spécifique est associée à un anticorps de détection et permet de faire varier la couleur de l'échantillon. L'élaboration d'un tel procédé nécessite donc de coupler un anticorps à une enzyme spécifique, avec le risque de dénaturer l'enzyme de détection.

Outre le domaine du diagnostique, le greffage de biomolécules sur un support solide est couramment utilisé dans les activités de recherche, telles que le screening de nouvelles molécules, impliquant souvent de très larges librairies d'échantillons.

Dans le domaine médical, le couplage de plusieurs molécules est souvent indiqué par exemple pour cibler une action thérapeutique particulière. Les peptides, polypeptides, protéines font ainsi souvent l'objet de fonctionnalisation chimique pour le développement de produits pharmacologiques. De telles fonctionnalisations permettent d'améliorer les effets thérapeutiques, ou d'atténuer les effets secondaires, ou de produire de nouveaux effets. Des biomolécules telles que des anticorps peuvent être fonctionnalisées avec une autre molécule thérapeutique de sorte à cibler précisément les cellules à traiter. Cette approche est notamment déployée en oncologie pour délivrer le traitement spécifiquement aux cellules cancéreuses et limiter ou éviter les effets secondaires. Cependant, la réaction de fonctionnalisation ou de couplage de telles biomolécules avec d'autres molécules peut se révéler complexe et peut modifier ou dénaturer les biomolécules. Le plus souvent, la molécule intermédiaire, faisant le lien entre les molécules, doit être spécifiquement développée pour les molécules à apparier, ce qui rend le travail fastidieux. En outre, il n'est pas toujours facile de contrôler dans quelle mesure le couplage est effectivement réalisé.

A des fins de diagnostique, de recherche et développement ou d'application thérapeutique, il apparaît nécessaire de développer un lien chimique utilisable aisément dans un grand nombre d'applications différentes de sorte à accélérer et simplifier la production de nouveaux dispositifs de test, de diagnostique ou des méthodes thérapeutiques.

L'intolérance au lactose représente un problème majeur pour l'alimentation des nourrissons. Le polymorphisme LCT-13910 C/T de l'intron 13 a été identifié comme responsable de l'intolérance lactose. Le diagnostique nécessite cependant des analyses en laboratoire. Il convient donc de développer un test de diagnostique facile à produire et à mettre en oeuvre pour caractériser rapidement une intolérance au lactose.

Il existe un réel intérêt à développer de nouveaux dérivés permettant en particulier de fonctionnaliser des peptides ou des protéines ou d'autres polymères biologiques tels que des acides nucléiques. Il existe en outre un réel intérêt pour de nouveaux agents de liaison permettant de fixer de tels polymères biologiques sur un support solide. Plus particulièrement, il apparaît nécessaire de développer de nouveaux dérivés susceptibles de permettre divers types d'applications, telles que des fonctionnalisations de peptides ou d'acides nucléiques ou leur greffage sur un support solide, ou comme agent révélateur dans un test diagnostique.

### Bref résumé de l'invention

Un but de la présente invention est de proposer un lien chimique adapté au greffage ou au couplage d'une biomolécule sur un support solide et/ou à une molécule conjuguée. En l'occurrence, il s'agit de proposer un lien chimique applicable à une multitude de biomolécules, incluant les peptides, protéines, acides nucléiques ou ribonucléiques, sans devoir être modifié ou impliquant peu de modifications. Il s'agit en outre de proposer un lien chimique ne nécessitant pas ou peu de modification des biomolécules à coupler ou greffer de sorte à ne pas les dénaturer.

Un autre but de la présente invention est de proposer un lien chimique permettant des couplages séquentiels et/ou alternatifs sur différents supports solides. En d'autres termes, un même lien chimique peut être utilisé pour le greffage de biomolécules sur différents types de supports. L'objectif est de produire un lien comportant plusieurs fonctions chimiques réactives pouvant être activées de manière indépendante pour permettre le couplage séquentiel et/ou alternatif.

Un autre but de la présente invention est de proposer un lien chimique adapté pour réagir spontanément avec un ou plusieurs sites réactifs d'une biomolécule de sorte à effectuer un coupage ou un greffage rapide et simple, dans des conditions de réactions douces. De préférence le lien chimique réagit de façon sélective sur un ou plusieurs sites prédéterminés.

Un autre but de la présente invention est de proposer un lien chimique permettant de visualiser aisément et instantanément l'établissement du couplage ou du greffage des molécules. Un autre but est de pouvoir doser ou suivre l'avancement du couplage de manière visuelle directe ou via un dispositif optique.

Un autre but de la présente invention est de proposer une méthode de production de biosimilaires, ainsi que des biosimilaires ainsi produits, au moyen de la fonctionnalisation de protéine ou d'anticorps avec un principe actif.

Un autre but de la présente invention est de proposer une méthode de production d'un dispositif de test ou de diagnostique impliquant un lien chimique. En l'occurrence, la méthode de production permet de greffer une ou plusieurs biomolécules sur un support solide et/ou de la coupler avec une autre molécule, de manière simple, rapide et peu couteuse.

Un autre but de la présente invention est de proposer un test d'identification d'un marqueur biologique, de façon simple, rapide et fiable, dans lequel au moins un des éléments du test est lié à un support solide. En particulier, la présente invention propose un test diagnostique de l'intolérance au lactose facile à mettre en oeuvre.

Un autre but de la présente invention est de proposer un test immunologique comprenant un indicateur coloré ne résultant pas d'une activité enzymatique. L'objectif est en particulier de proposer un test immunologique ayant un indicateur coloré fiable, robuste et facile à mettre en œuvre.

Un autre but de la présente invention est de proposer un kit comprenant un test diagnostique et un lien de greffage et/ou de couplage d'un biosenseur sur un support solide.

Un autre but de la présente invention est de proposer une méthode de couplage d'une biomolécule avec une autre molécule, en particulier une molécule présentant une activité thérapeutique. Il s'agit également de proposer une méthode ou un procédé de fonctionnalisation de peptides, de protéines ou d'acides nucléique ou ribonucléique.

Un autre but de l'invention est de proposer une méthode ou un procédé de greffage d'un biosenseur sur une surface solide de façon fiable, robuste et simple.

Un autre but de la présente invention est de proposer une méthode de captage de certaines biomolécules ou catégories de biomolécules via l'immobilisation de peptides, de protéines ou d'acides nucléiques sur un support solide. Avantageusement le captage d'un peptide ou d'un acide nucléique permet en outre un dosage instantané, de préférence via une indication visuelle directe ou identifiable par un appareil optique.

Un autre but de la présente invention est de proposer une méthode de fonctionnalisation d'une surface solide via l'immobilisation de peptides, de protéines ou d'acides nucléiques. En particulier, l'objectif est de rendre réactive une telle surface à un stimulus particulier, notamment un stimulus lumineux. Un objectif est de fonctionnaliser une surface solide avec des cryptochromes de sorte à devenir photosensible.

Selon l'invention, ces buts sont atteints notamment au moyen des dérivés de dicyanomethanide de phthalazimium de formule (I) décrits dans les revendications, utilisés comme liens pour le greffage de biomolécules sur une surface solide ou pour lien de couplage avec d'autres molécules. Ces buts sont également atteints au moyen des dispositifs de test et/ou de diagnostique impliquant l'utilisation de dérivés de dicyanomethanide de phthalazimium de formule (I). Ces buts sont également atteints au moyen d'un test de diagnostique de type PLISA (Polymer-Linked Immuno assay) comprenant un polymère pourvu d'un ou plusieurs sites réactifs aux dérivés de dicyanomethanide de phthalazimium de formule (I).

L'invention représente notamment l'avantage de proposer un lien applicable à différentes applications avec peu ou pas de modifications chimiques. L'invention permet de dériver des peptides et acides nucléique, ou de greffer des surfaces solides via des liaisons covalentes par des réactions «one-pot». L'invention permet d'immobiliser localement des biosenseurs sur une surface solide de sorte à produire des tests multiplexes. Les spots sont limités à des surfaces de l'ordre de 100 µm ou 50 µm. L'inventine permet de fonctionnaliser des surfaces solides avec une monocouche de biomolécules. L'invention permet de produire des tests et des diagnostiques avec des quantités restreintes de matériel biologique greffé sur la surface solide, de l'ordre de 10 à 50 fois moins que pour les méthodes déjà connues.

### Brève description des figures

La présente invention est illustrée par les figures suivantes :

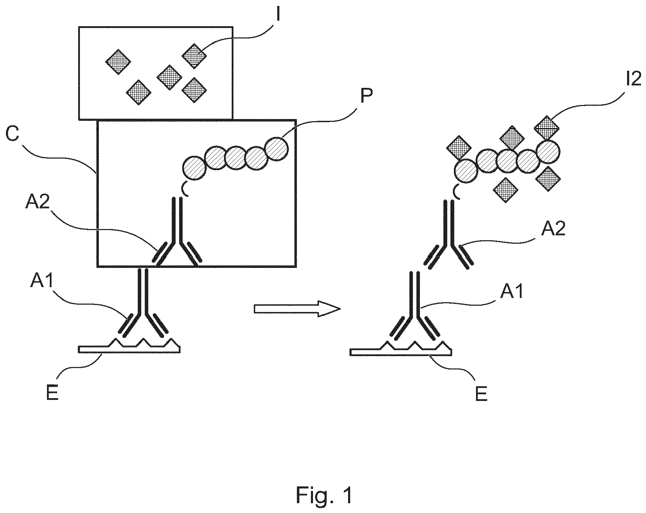

- Figure 1 : représentation schématique d'une application de test selon un exemple de la présente invention.

### Exemple(s) de mode de réalisation de l'invention

Pour les besoins de la présente invention un lien chimique désigne une molécule organique comprenant au moins un site fonctionnel permettant de lier de manière covalente cette molécule organique à une autre molécule, en particulier une biomolécule, une molécule à tester ou faisant office de biosenseur. Le site fonctionnel peut être en l'occurrence un groupement dicyanométhanide. La liaison covalente peut être établie par toute réaction chimique connue dans des conditions de réaction suffisamment douces pour ne pas dénaturer la biomolécule ou le biosenseur visé. En particulier, la réaction s'effectue sans chauffage ou avec un chauffage modéré. La réaction s'effectue dans un milieu aqueux et/ou sans solvent organique. La réaction est de préférence spontanée. La réaction est de préférence sélective d'un ou plusieurs sites prédéterminés de la biomolécule à greffer ou du biosenseur. La liaison covalente peut être établie via une cycloaddition. Le site de réaction prédéterminé de la biomolécule peut comporter une insaturation, telle qu'une double liaison, susceptible de réagir dans une cycloaddition. De préférence le site de réaction prédéterminé de

La molécule formant le lien chimique selon la présente invention comporte avantageusement un ou plusieurs autres sites fonctionnels permettant de lier la molécule organique sur une surface solide ou sur une autre molécule. En particulier, des surfaces de silicium ou de verre, comportant des restes - SiOH, peuvent être utilisées à cette fin. Des surfaces de produits synthétiques comportant des sites réactifs tels que des insaturations peuvent alternativement être utilisées. Par exemple, des supports à base d'acrylonitrile de styrène et de butadiène, ABS (Acrylonitrile Butabiène Styrene) peuvent être utilisés. La molécule organique faisant office de lien comporte alors une ou plusieurs fonctions chimiques susceptibles d'interagir avec l'une ou plusieurs de ces fonctions de ces surfaces solides. Les fonctions chimiques de la molécule organique servant de lien peuvent être libres ou bien protégées de sorte à être chimiquement libérées in-situ. Quelle comporte ou non des fonctions chimiques permettant son greffage sur une surface solide, la molécule organique servant de lien peut être utilisée en solution sans être nécessairement greffée sur un support solide.

Selon une disposition avantageuse, la molécule organique faisant office de lien peut produire un effet visuel lors de sa réaction avec une molécule à tester ou une biomolécule. En particulier, la molécule organique servant de lien, lorsqu'elle est en solution, peut colorer la solution avec une intensités liée à sa concentration, ou directement proportionnelle à sa concentration. La coloration due à la molécule organique faisant office de lien peut varier ou disparaître lorsqu'elle a réagi avec une biomolécule ou une molécule à tester, ou une surface solide. Avantageusement, la variation ou la disparition de couleur peut être avantageusement utilisée pour suivre l'avancement d'une réaction de couplage ou de greffage. Dans le cas où la molécule organique réagit sur une biomolécule ou une molécule à tester, la variation ou la disparition de couleur qui en résulte peut permettre de doser la biomolécule ou molécule à tester.

Les termes de surface solide ou de support, et tout équivalent, désignent indistinctement tout matériau solide susceptible de recevoir et de maintenir des molécules organiques servant de lien avec d'autres molécules ou des biomolécules. Selon les besoins, une surface solide peut prendre la forme d'une plaque aux dimensions macroscopiques, permettant notamment d'effectuer un test visuel ou colorimétrique. Alternativement une surface solide peut être constituée d'un ensemble de microbilles, également connues sous le terme anglais de « microbeads ». Alternativement, une surface solide peut être un matériau poreux offrant une large surface de contact avec le milieu extérieur. Une surface solide peut désigner alternativement un ensemble de fibres textiles ou synthétiques. Une surface peut être faite de, ou comporter, la cellulose. Il est entendu que le support ou la surface solide ou tout terme équivalent désigne un élément hors solution, même en présence d'une solution.

Le terme de biomolécule désigne toute molécule d'intérêt biologique ou issue du monde vivant, en particulier des macromolécules de type peptide, protéine, anticorps, enzyme, hormone, récepteur biologique, récepteur transmembranaire, acide nucléique, acide ribonucléique, acide désoxyribonucléique, ainsi que leur dérivés, sels, et combinaisons. Les biomolécules peuvent être fonctionnalisées de sorte à comporter des portions non naturelles. Les biomolécules peuvent en outre désigner des acides gras, acides aminés, neurotransmetteurs. De préférence, le terme de biomolécule désigne une molécule du monde vivant à tester ou identifier au moyen des produits et méthodes de la présente invention.

Les termes de molécule à tester désigne toute molécule dont la présence dans un échantillon doit être identifiée au moyen des produits et méthodes de la présente invention. Une molécule à tester peut être une biomolécule telle que définie ci-dessus. Alternativement une molécule à tester peut être ou comprendre une molécule synthétique comportant au moins un site réactif permettant de l'identifier. Plus particulièrement, une molécule à tester peut être un polymère, tel qu'un acide polyacrylique, comportant au moins une fonction réactive avec le lien décrit ci-dessus, notamment un acide polyacrylique de plus de 200 ou 300 ou 400 ou 450 KDa fonctionnalisé avec des allyls.

Le terme de biosenseur désigne toute molécule adaptée à s'apparier de manière spécifique avec une biomolécule ou une molécule à tester dans les conditions de la présente description. Un biosenseur peut en l'occurrence comporter un ou plusieurs épitopes. Un biosenseur peut prendre la forme d'une séquence d'ADN ou d'ARN pouvant s'apparier avec une séquence complémentaire d'une biomolécule ou d'une molécule à tester. Alternativement, un biosenseur peut prendre la forme d'un peptide ou d'une protéine dont la séquence peut être reconnue par un anticorps. Un biosenseur peut être également désigné sous le terme de biocapteur.

### Molécule organique

La molécule organique utilisée comme lien est représentée par les dérivés de dicyanomethanide de phthalazimium de formule (I) 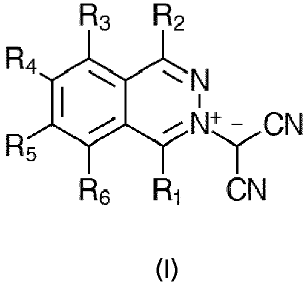 dans laquelle les substituants R¹, R², R³, R⁴, R⁵, et R⁶ désignent indépendamment l'un de l'autre un atome ou groupe d'atomes sélectionné parmi le groupe consistant en
- SO₃⁻, PO₄, NO₂, -NHS
- -(CH₂)ₓ-OR⁷, -(CH₂)ₓ-OH, -(CH₂)ₓ-NR⁷R⁸, -(CH₂)ₓ-NHR⁸, -(CH₂)ₓ-CONR⁷R⁸, - (CH₂)ₓ-CONHR⁸, -(CH₂)ₓ-CONH₂, -(CH₂)ₓ-CO₂NR⁷R⁸, -(CH₂)ₓ-CO₂NHR⁸, - (CH₂)ₓ-COR⁷, -(CH₂)ₓ-COH, -(CH₂)ₓ-COOR⁷, -(CH₂)ₓ-COOH, -(CH₂)ₓ-SR⁷, - (CH₂)ₓ-SH, -(CH₂)ₓ-SOR⁷, -(CH₂)ₓ-SOH, -(CH₂)ₓ-SO₂R⁷, -(CH₂)ₓ-SO₂H où x est un nombre entier compris entre 0 et 10,
- un atome d'halogène, et
- R⁷

où R⁷ désigne un atome ou groupe d'atomes sélectionné parmi le groupe consistant en
   - un alkyl linéaire ou ramifié comprenant de 1 à 12 atomes de carbone, pouvant être saturé, mono-insaturé ou poly-insaturé, dans lequel 1 ou plusieurs atomes d'hydrogène peuvent être indépendamment l'un de l'autre remplacés par un atome d'halogène, un groupement -COR¹⁰, -CO₂R¹⁰, -OR¹⁰, -NO₂, NHR¹⁰, ou un groupement phényl éventuellement substitué par 1, 2, 3 ou 4 groupements indépendamment sélectionnés dans le groupe consistant en -COR¹⁰, -CO₂R¹⁰, -OR¹⁰, -NO₂, -NHR¹⁰, N3,
   - un cycloalkyl comprenant 4, 5, 6, 7, 8 ou 9 atomes de carbones, pouvant être saturé, mono-insaturé, doublement ou triplement insaturé, dans lequel 1 ou plusieurs atomes d'hydrogène peuvent être indépendamment l'un de l'autre remplacés par un atome d'halogène, un groupement -COR¹⁰, -CO₂R¹⁰, -OR¹⁰, - NO₂, NHR¹⁰, ou un groupement phényl éventuellement substitué par 1, 2, 3 ou 4 groupements indépendamment sélectionnés par le groupe consistant en -COR¹⁰, -CO₂R¹⁰, -OR¹⁰, -NO₂, NHR¹⁰ et
   - un groupement aromatique comprenant 1 ou 2 cycles aromatiques éventuellement substitué par 1, 2, 3 ou 4 groupements indépendamment sélectionnés par le groupe consistant en -COR¹⁰, -CO₂R¹⁰, -OR¹⁰, -NO₂, NHR¹⁰, N³, -CH(CN)₂,
où R⁸ désigne un atome ou groupe d'atomes sélectionné parmi le groupe consistant en
   un atome d'hydrogène et
   R₇,
   ou bien dans laquelle R⁸ forme avec R⁷ dans les groupes -(CH₂)ₓ-NR⁷R⁸, -(CH₂)ₓ-CONR⁷R⁸, -(CH₂)ₓ-CO₂NR⁷R⁸, un hétérocycle comprenant de 4, 5, 6 ou 7 atomes de carbone, où 1 ou 2 groupes -CH₂- peuvent être indépendamment l'un de l'autre remplacés par -CO-, ou -CS-, et où deux atomes de carbone contigus peuvent être liés par une double liaison ;
où R¹⁰ désigne un atome d'hydrogène ou un groupe alkyl saturé comprenant de 1 à 4 atomes de carbone ;
où au moins l'un des substituants R¹, R², R³, R⁴, R⁵, et R⁶ est différent de H, ou bien si l'un des 6 substituants R¹, R², R³, R⁴, R⁵, et R⁶ est un atome de chlore, ou un groupe -OCH₃, -SCH₃, -CF₃, ou phenyl, alors au moins l'un des 5 autres substituants est différent de H, ou si 2 des substituants R¹, R², R³, R⁴, R⁵, et R⁶ sont des groupes -CH₃, alors au moins l'un des 4 autres substituants est différent de H.

Selon un mode de réalisation particulier, le substituant R⁶ désigne SO₃⁻, PO₄, NHS ou NO₂,
- les substituant R² et R⁵ désignent indépendamment l'un de l'autre un atome ou un groupe d'atomes sélectionné dans le groupe consistant en un atome d'hydrogène, NHS, -(CH₂)ₓ-COOR⁷, -(CH₂)ₓ-COOH, -(CH₂)ₓ-CO₂NR⁷R⁸, où x est un nombre entier correspondant à 0 ou 1,
- les substituants R³ et R⁴ désignent indépendamment l'un de l'autre un atome ou un groupe d'atomes sélectionné dans le groupe consistant en un atome d'hydrogène, un atome d'halogène, -OR¹⁰ et -COR¹⁰,

où R⁷ désigne un atome ou groupe d'atomes sélectionné parmi le groupe consistant en un alkyl linéaire ou ramifié comprenant de 1 à 12 atomes de carbone, pouvant être saturé, mono-insaturé ou poly-insaturé, dans lequel 1 ou plusieurs atomes d'hydrogène peuvent être indépendamment l'un de l'autre remplacés par un atome d'halogène, un groupement-COR¹⁰, -CO₂R¹⁰, -OR¹⁰, -NO₂, -NHR¹⁰, ou un groupement phényl éventuellement substitué par 1, 2, 3 ou 4 groupements indépendamment sélectionnés par le groupe consistant en -COR¹⁰, -CO₂R¹⁰, -OR¹⁰, -NO₂, -NHR¹⁰,
ou bien dans laquelle R⁸ forme avec R⁷ dans le groupe -(CH₂)ₓ-CO₂NR⁷R⁸, un hétérocycle comprenant de 4, 5, ou 6 atomes de carbone, où 1 ou 2 groupes -CH₂- peuvent être indépendamment l'un de l'autre remplacés par -CO-, ou -CS-, et où deux atomes de carbone contigus peuvent être liés par une double liaison ;
où R¹⁰ désigne un atome d'hydrogène ou un groupe alkyl saturé comprenant de 1 à 4 atomes de carbone ;

Selon un autre mode de réalisation, le substituant R¹ désigne un atome d'hydrogène,
- le substituant R⁶ désigne SO₃⁻ ou PO₄,
- les substituants R² et R⁵ désignent indépendamment l'un de l'autre un atome ou un groupe d'atomes sélectionné dans le groupe consistant en un atome d'hydrogène, NHS,-COOR⁷, -COOH, - CO₂NR⁷R⁸,
- les substituants R³ et R⁴ désignent indépendamment l'un de l'autre un atome ou un groupe d'atomes sélectionné dans le groupe consistant en un atome d'hydrogène, un atome de chlore, et un atome de fluor,
   où R⁷ désigne un atome ou groupe d'atomes sélectionné parmi le groupe consistant en un alkyl saturé linéaire ou ramifié comprenant de 1 à 6 atomes de carbone, dans lequel 1 ou plusieurs atomes d'hydrogène peuvent être indépendamment l'un de l'autre remplacés par un atome d'halogène, un groupement -COR¹⁰, -CO₂R¹⁰, -OR¹⁰, -NO₂, -NHR¹⁰,
ou bien dans laquelle R⁸ forme avec R⁷ dans le groupe -CO₂NR⁷R⁸, un hétérocycle comprenant de 4, ou 5 atomes de carbone, où 1 ou 2 groupes - CH₂- peuvent être indépendamment l'un de l'autre remplacés par -CO-, ou - CS-, et où deux atomes de carbone contigus peuvent être liés par une double liaison ;

De manière privilégiée, les dérivés de dicyanométhanide de phthalazinium sont sélectionnés parmi les composés suivants :

| | | | |
|---|---|---|---|
| P1 | 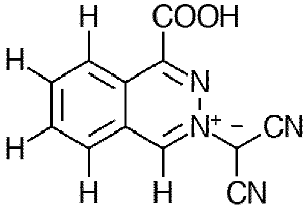 | P2 | 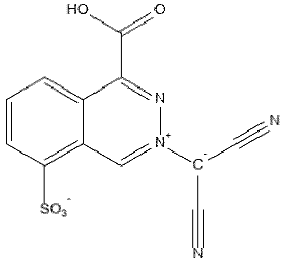 |
| P3 | 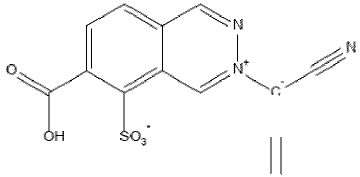 | P4 | 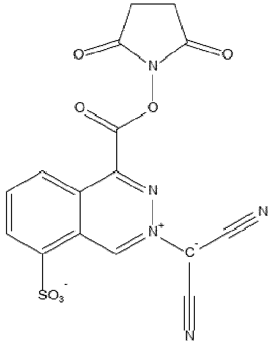 |
| P5 | 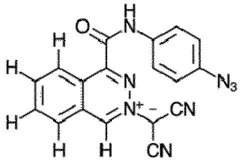 | P6 | 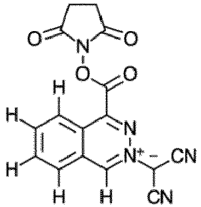 |
| P7 | 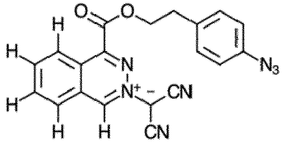 | P8 | 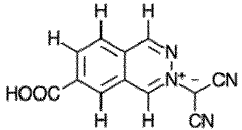 |
| P9 | 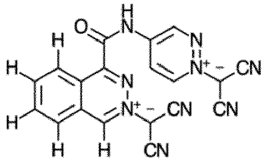 | P10 | 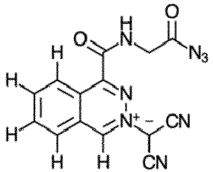 |
| P11 | 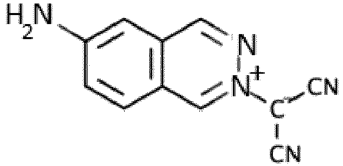 | P12 | 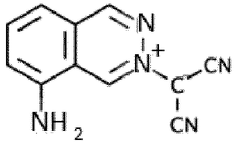 |

Les composés de formule (I) peuvent être préparés selon le procédé de production décrit ci-après. Les dérivés de dicyanométhanide de phthalazinium (I) sont produits à partir de dérivés de la phthalazine. En particulier, le composé de formule (Ib), dans lequel les substituants R¹, R², R³, R⁴, R⁵ et R⁶ sont tels que décrits plus haut, est mis en réaction avec l'oxyde de tétracyanoéthylène (TCNEO) selon le schéma suivant :

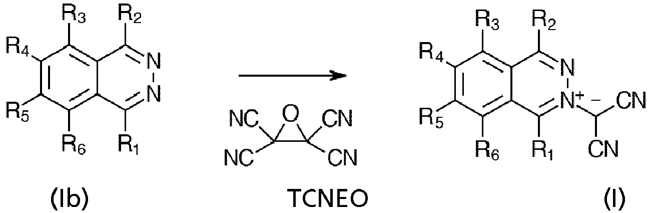

Les composés obéissant à la formule (Ib) dans lesquels R² désigne un groupement -(CH₂)ₓ-COOH où x est égal à 0 et où R¹, R³, R⁴, R⁵ et R⁶ sont tels que défini plus haut, sont obtenus à partir de dérivés de phthalazine où R²=H selon les réactions suivantes : 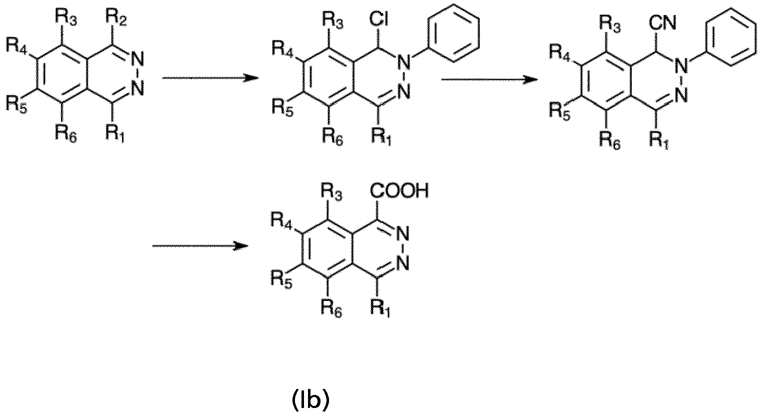 Alternativement, les composés obéissant à la formule (Ib) dans lesquels R² désigne un groupement -(CH₂)ₓ-COOH où x est égal à 0 et où R¹, R³, R⁴, R⁵ et R⁶ sont tels que défini plus haut, peuvent être obtenus selon les réactions suivantes :

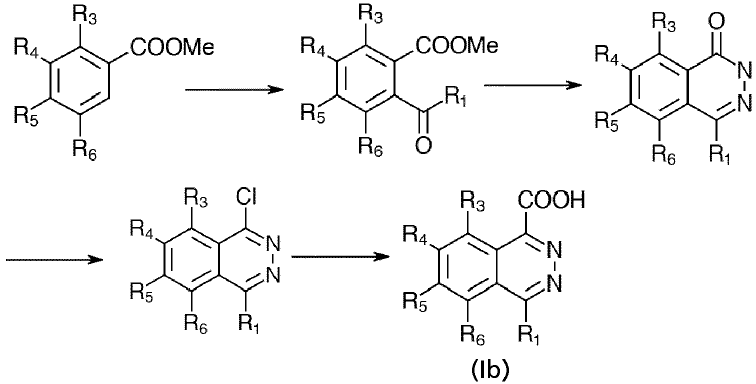

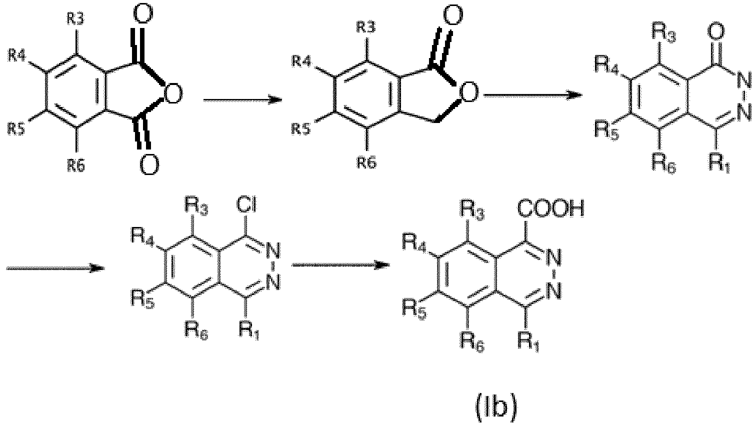

### Fonctionnalisation et/ou greffage de peptides et protéines

La présente invention couvre également une méthode de fonctionnalisation d'une biomolécule de type peptides et/ou protéines (PT), incluant les enzymes et les anticorps, ou leur greffage sur un support solide. A cet effet, les composés de formule (I) sont mis en réaction avec un peptide ou une protéine comprenant au moins un tryptophane, de sorte à réagir avec le groupement indole du tryptophane. Le peptide ou la protéine modifiée (III) peut alors être obtenue :

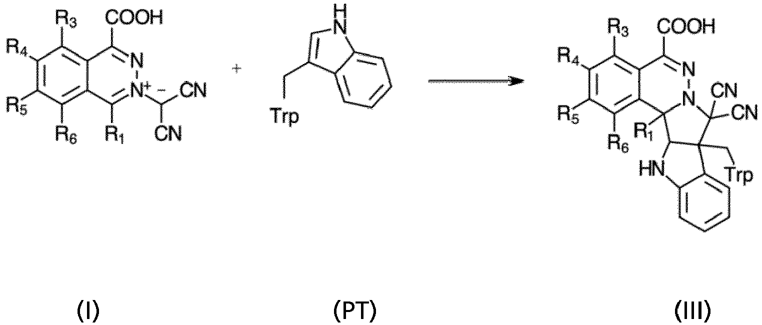

Selon un mode de réalisation, tous les sites d'une telle biomolécule accessibles au composé de formule (I) peuvent être fonctionnalisés de manière identique, avec un composés de formule (I) dans lequel tous les groupes R¹, R², R³, R⁴, R⁵ et R⁶ sont identiques.

Selon un autre mode de réalisation, les sites de la biomolécule sont fonctionnalisés par deux ou plus de deux composés de formule (I) dont au moins l'un des groupes R¹, R², R³, R⁴, R⁵ et R⁶ est différent.

L'un des groupements R¹, R², R³, R⁴, R⁵ et R⁶ peut comporter un groupe réactionnel permettant de fixer la biomolécule fonctionnalisée sur un support solide. Par exemple, des supports solides tels que le verre, la cellulose ou des polymères d'acrylonitrile de butadiène et de styrène (ABS ; Acrylonitrile Butadiene Styrene) peuvent être utilisés. Selon les besoins, un ou plusieurs des groupes R³, R⁴ ou R⁶ peuvent être dérivés avec une amine. Dans le cas d'un support en verre, un ou plusieurs des groupes R⁴ ou R⁵ peuvent être dérivés par un silane de sorte à réagir avec la surface du verre.

Les composés de formule (I) peuvent alternativement être mis en réaction avec le styrène (ST), pour conduire aux composés de formule (IV) suivant la réaction suivante :

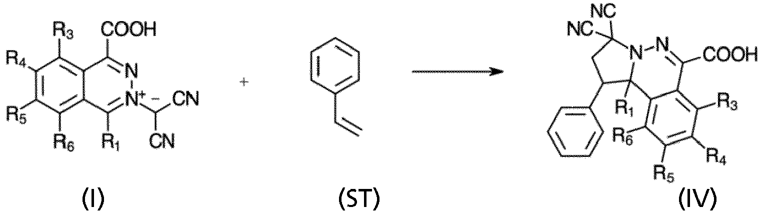

La double liaison du styrène sur laquelle réagit le composant de formule (I) peut être l'extrémité libre d'un support solide comprenant du polystyrène. En particulier, des microbilles de polystyrène peuvent être utilisées comme support solide. L'un ou plusieurs des groupes R³, R⁴, R⁵, R⁶ peuvent être adaptés pour réagir avec une biomolécule ou une molécule à tester.

De cette manière, un peptide ou une protéine peut être fixée de manière covalente sur le support au moyen du lien décrit ci-dessus via une réaction de cycloaddition. Dans le cas où la fonction dicyanométhanide de la molécule de formule (I) réagit sur un peptide ou une protéine, la réaction est spécifique du tryptophane, seul acide aminé pourvu d'une double liaison susceptible de réagir avec le lien.

Des acides nucléiques tels que des oligomères d'ADN ou d'ARN peuvent également être fixés sur un support solide en utilisant le lien décrit ici. Dans le cas d'un oligomère d'ADN, la molécule de formule (I) peut être mise en réaction avec une ou plusieurs des bases thymines comprises dans l'ADN. Dans le cas de l'ARN, la molécule de formule (I) peut être mise en réaction avec une ou plusieurs des bases uracyl comprises dans l'ARN.

Les réactions décrites ici, telles que la réaction des produits de formule (I) avec le styrène, le tryptophane, la thymine et l'uracile, peuvent être effectuées dans des conditions thermiques usuelles connues de l'homme de métier. Alternativement, une irradiation lumineuse peut être utilisées selon les besoins, telles qu'une irradiation UV ou UV-visible.

Dans le cas où la fonction dicyanométhanide réagit avec le support solide, par exemple sur un groupe styrène, l'un ou plusieurs des groupes R³, R⁴, R⁵, R⁶ peut être impliqué dans une réaction avec une biomolécule ou une molécule à tester. Tout type de réaction peut alors être envisagée, qu'elle soit sélective ou non d'un site particulier de la biomolécule ou de la molécule à tester.

Sur un support solide donné, plusieurs zones peuvent être définies et un composé de formule (I) différent peut être greffé dans chacune des zones. En l'occurrence, lorsque la fonction dicyanométhanide réagit avec le support solide, au moins l'un des groupes R¹, R², R³, R⁴, R⁵ et R⁶ peut varier d'une zone à l'autre de sorte à réagir avec différentes biomolécules. Ainsi, plusieurs biomolécules se trouvent greffées sur un support.

Alternativement, le lien de formule (I) peut être utilisé pour le couplage d'une biomolécule avec un une autre molécule selon les mêmes dispositions que décrit précédemment. Dans ce cas, la fonction dicyanométhanide réagit avec une base thymine, une base uracyl ou un tryptophane d'une biomolécule ou d'une molécule à tester et l'un ou plusieurs des groupes R³, R⁴, R⁵, R⁶ réagissent avec l'autre molécule à coupler à la biomolécule. La molécule couplée peut être de du type peptide, protéine ou acide nucléique. La molécule couplée peut alternativement être une molécule synthétique telle qu'un polymère, éventuellement fonctionnalisé. Un tel polymère peut être par exemple un acide polyacrylique fonctionnalisé avec des allyls.

La situation inverse est bien entendu également possible, dans laquelle la fonction dicyanométhanide réagit avec la molécule à coupler et l'un des groupes R¹, R², R³, R⁴, R⁵ et R⁶ réagit avec une biomolécule ou une molécule à détecter.

Selon un mode de réalisation particulier, l'un des groupes R¹, R², R³, R⁴, R⁵ et R⁶ comporte une fonction dicyanométhanide de sorte que le lien de formule (I) comporte deux fonctions dicyanométhanide. Ainsi, le lien de formule (I) peut se coupler sélectivement à une base thymine, uracile, un tryptophane ou un styrène via l'une de ses fonctions dicyanométhanide et sélectivement a une base thymine, uracile, un tryptophane d'une autre molécule via sa seconde fonction dicyanométhanide.

Selon un mode de réalisation, la molécule de formule (I) est en solution de sorte à réagir avec la surface solide ou une biomolécule ou une molécule à tester également en solution. Le composé de formule (I), lorsqu'il est libre, induit une couleur donnée à la solution, qui disparaît ou change lorsqu'il réagit avec la surface solide ou la biomolécule ou la molécule à tester. Le greffage du lien sur la surface ou la fonctionnalisation de biomolécules ou de molécules à tester peut alors être suivi visuellement. L'avancement ou la complétude de la réaction peut être déterminé via un dispositif optique ou en comparaison avec une échelle de couleur préalablement déterminée.

### Test diagnostique à base d'un oligomère d'acide nucléique et test d'intolérance au lactose

Selon un mode de réalisation, le diagnostic selon la présente description comprend une étape a) de fixation du composé (I) de manière covalente sur un support solide. La fixation s'effectue dans les conditions décrites plus haut.

Selon un mode de réalisation, la fonction dicyanométhanide du composé de formule (I) est mise en réaction avec une insaturation du styrène d'un support comprenant du polystyrène, en solution, dans des conditions thermiques usuelles.

Le test comporte une étape b) de réaction du composé de formule (I) avec un oligomère d'acide nucléique à greffer sur le support solide.

Selon un mode de réalisation, un des groupements R¹, R², R³, R⁴, R⁵ o R⁶ de préférence R⁴ ou R⁵, est mis en réaction avec l'acide nucléique à fixer sur le support solide. Les deux étapes a) et b) peuvent être conduites dans cet ordre ou peuvent être inversées en fonction des besoins.

Le test comporte une étape c) de reconnaissance de l'oligomère d'acide nucléique fixé sur le support solide par un oligomère complémentaire. Selon un mode de réalisation, l'oligomère complémentaire est simplement incubé sur le support greffé à une concentration de 0.5 uM dans un tampon physiologique (phosphate buffered saline) pendant 30 min à température ambiante. Ensuite, le support est rincé avec le tampon phosphate pour retirer l'excédant d'oligomère complémentaire.

Le test comporte en outre une étape d) d'amplification permettant d'augmenter le signal à détecter.

Le test comporte une étape finale e) de détection permettant de déterminer si l'oligomère complémentaire s'est effectivement fixé sur l'oligomère immobilisé sur le support solide.

Selon un mode de réalisation particulier, le test concerne un test diagnostic d'intolérance au lactose. A cet effet, une séquence de peptide acide nucléique (peptide nucleic acid (PNA)) complémentaire au polymorphisme LCT-13910 C/T de l'intron 13 du gène MCM6 est fixé sur un support en cellulose via l'un des groupe R¹, R², R³, R⁴, R⁵ o R⁶ du composant (I) au moyen d'une fonction chimique photosensible. La séquence de peptide acide nucléique est alors greffée via la fonction dicyanométhanide du lien de formule (I). Un échantillon de sang du patient est prélevé et déposé sur le support solide comprenant la séquence peptide acide nucléique de sorte à vérifier la présence ou non d'une séquence complémentaire. Une étape d'amplification connue sous l'acronyme LAMP est effectuée et le test est lu.

### Kit et test diagnostique

La présente description couvre la méthode de test décrite ci-dessus et les éléments du test diagnostiques. Les éléments du test diagnostique peuvent être proposés sous forme de kit comprenant au moins un support solide. Plusieurs supports solides comprenant des matériaux différents tels que du verre, de la cellulose ou un polystyrène, peuvent être inclus et utilisables pour différentes applications. Alternativement ou en plus, un support solide peut comporter plusieurs zones prédéfinies et adaptées pour greffer différents type de molécules.

Le kit comprend au moins une dose d'un produit de formule (I). Plusieurs doses peuvent être présentes, soit toutes identiques, soit comportant différentes quantités de produits de formule (I). Dans le cas où plusieurs doses sont présentes, elles peuvent contenir toutes le même produit de formule (I). Alternativement, une sélection de plusieurs produits de formule (I) différents peut être proposée, dans lesquels au moins un des groupes R¹, R², R³, R⁴, R⁵ o R⁶ varie. Chacun des produits de formule (I) peut être préconisé pour une ou plusieurs applications spécifiques. Selon un mode de réalisation, les différents produits de formule (I) sont utilisés comme tests précurseur pour sélectionner le meilleur d'entre eux pour un opérer un diagnostique spécifique.

Le ou les produits de formule (I) peuvent être présentés sous toute forme adéquate, telle que sous forme de poudre à solubiliser ou déjà en solution.

Le kit comporte au moins un biosenseur spécifique d'une biomolécule ou d'une molécule à tester. Plusieurs biosenseurs peuvent être proposés selon les besoins, pouvant être indépendamment sélectionnés parmi un peptide, une protéine, une enzyme, un anticorps, un acide ribonucléique, un acide désoxyribonucléique, ou tout équivalent fonctionnalisé ou additivé.

Selon les besoins, le kit de test peut comporter un ou plusieurs produits tels que des anticorps, enzyme, ou indicateur coloré permettant d'identifier et éventuellement doser une biomolécule ou une molécule à tester.

Le kit peut en outre comprendre un ou plusieurs accessoires tels que pipettes, vials ou récipients permettant de mettre en pratique le test.

Le kit peut en outre comporter une notice ou des indications techniques à l'attention de l'utilisateur et permettant de mettre en oeuvre le diagnostique et/ou d'en lire le résultat.

Selon un mode de réalisation particulier, le kit selon la présente description comporte au moins un support solide dont la surface est pourvue de fonctions chimiques réactives adéquates, au moins une dose d'un produit de formule (I) pouvant réagir avec les fonctions chimiques du support et au moins un biosenseur comprenant au moins une fonction chimique pouvant réagir avec le composé de formule (I), où le composé de formule (I) est utilisé comme lien pour greffer le biosenseur sur le support. Le biosenseur peut être en particulier une séquence de peptide acide nucléique (peptide nucleic acid (PNA)) complémentaire au polymorphisme LCT-13910 C/T de l'intron 13 du gène MCM6.

Selon un autre mode de réalisation, le kit selon la présente description comporte au moins un support solide et un biosenseur greffé ou pouvant être greffé sur le support solide, au moins une dose d'un produit de formule (I) en solution ou pouvant être mis en solution et au moins un complexe révélateur pouvant réagir avec le composé de formule (I). En l'occurrence, le complexe révélateur peut être un anticorps primaire ou secondaire fonctionnalisé avec un polymère comprenant une ou plusieurs fonctions adaptées à réagir avec le composé de formule (I), en particulier avec la fonction dicyanométhanide du composé de formule (I) en solution. Par exemple, le complexe révélateur peut être un anticorps fonctionnalisé avec un acide polyacrylique comprenant des fonctions allyl accessible au produit de formule (I).

### Utilisation du produit de formule (I) comme révélateur

La figure 1 schématise un test PLISA dans lequel un composé de formule (I) selon la présente description est utilisé en solution comme indicateur coloré. Un biosenseur comprenant un épitope E est fixé sur une surface solide. Il peut être fixé par tout moyen déjà connu. Alternativement, le biosenseur peut être fixé au support solide au moyen d'un composé de formule (I), comme décrit ici. Un anticorps de détection, fonctionnalisé avec un polymère révélateur P, est associé à l'épitope E soit directement soit indirectement. Un composé de formule (I) en solution est mis en contact avec le polymère révélateur de sorte à réagir avec lui. La réaction du composé de formule (I) avec le polymère révélateur P s'accompagne d'une variation ou d'une disparition de la couleur de la solution. La couleur de la solution peut être visible directement ou dans le domaine de l'UV. Elle peut être mesurable par comparaison avec une échelle de couleur préétablie ou au moyen d'un dispositif optique adapté.

Selon un mode de réalisation, un anticorps primaire A1 est apparié à l'épitope E et un complexe C formé d'un anticorps secondaire A2 fonctionnalisé avec un polymère P est associé à l'anticorps primaire A1 comme illustré dans la figure 1. Selon un autre mode de réalisation, un complexe C formé d'un anticorps fonctionnalisé par un polymère révélateur Pest directement apparié à l'épitope E. Le polymère fonctionnalisé peut être par exemple un acide polyacrylique pourvu de une ou plusieurs fonctions allyl accessible au produit de formule (I), en particulier à sa fonction dicyanométhanide.

La méthode de test comporte ainsi une étape a') d'apparier un complexe C de détection formé d'un anticorps primaire A1 ou secondaire A2 fonctionnalisé avec un polymère de révélation P à l'épitope E d'un biosenseur fixé sur une surface solide.

La méthode comporte une étape b') de mettre en solution un composé de formule (I) de sorte à le faire réagir avec le polymère de révélation P. Le composé de formule (I) peut être présent dans la solution à une concentration prédéterminée. Le composé de formule (I) peut induire une coloration de la solution. La couleur de la solution peut changer ou disparaître au cours de la réaction du composé de formule (I) avec le polymère de révélation P.

La méthode peut comporter une étape c') de détection optique ou visuelle de la coloration de la solution de sorte à suivre la réaction et/ou doser la quantité de composé de formule (I) ayant réagi avec le polymère de révélation P.

La méthode PLISA peut comporter une étape préalable d'étalonnage de la coloration en fonction de la concentration du composé de formule (I).

### Elaboration d'un test de diagnostique

La présente description porte en outre sur l'élaboration rapide et simple d'un test sur support solide sur la base d'un composé ou d'un ensemble de composés de formule (I). De préférence le ou les composés de formule (I) sont déjà disponibles et n'ont pas été produits spécifiquement pour l'occasion.

L'élaboration d'un tel test comprend une étape a") d'évaluation d'un ou plusieurs composés de formule (I) dans une réaction de couplage avec un biosenseur déterminé ou avec une biomolécule ou avec une molécule à tester. Selon un mode de réalisation, le biosenseur est un peptide ou une protéine comprenant au moins une insaturation libre d'un tryptophane, un acide déoxyribonucléique comprenant au moins une base thymine libre ou un acide ribonucléique comportant au moins une base uracile libre, et mis en réaction avec la fonction dicyanométhanide du ou des composé de formule (I). Selon un autre mode de réalisation, la molécule à tester comprend un polymère P comportant au moins une fonction chimique propre à réagir avec au moins l'un des groupes R¹, R², R³, R⁴, R⁵, R⁶ ou dicyanométhanide du composé de formule (I). Par exemple, le polymère P peut être un acide polyacrylique fonctionnalisé avec des groupements allyl. De préférence, le groupe dicyanométhanide réagit avec les fonctions allyl du polymère P.

L'étape d'évaluation peut comprendre une étape a1") de détermination de l'avancement ou la complétude de la réaction, par exemple au moyen d'une mesure colorimétrique ou par UV. De préférence, plusieurs composés de formule (I) sont utilisés, dans lesquels au moins un des groupes R¹, R², R³, R⁴, R⁵ ou R⁶ varie. Les composés de formule (I) permettant les réactions les plus complètes ou les plus rapides peuvent être sélectionnés. D'autres critères que la cinétique ou le rendement de la réaction peuvent être pris en considération.

Selon un mode de réalisation, plusieurs des groupes R¹, R², R³, R⁴, R⁵ ou R⁶ sont adaptés à réagir sur un support spécifique tel que le verre, la cellulose ou le polystyrène. Ils peuvent être indépendamment les uns des autres libres ou bien protégés par un groupe protecteur. Dans ce dernier cas, une étape préalable d'activation de l'un ou plusieurs de ces groupes, par déprotection par exemple, doit être prévue si les composés doivent être greffés sur un support. De la sorte, les composés de formule (I), une fois couplés au biosenseur, peuvent être greffés sur différents supports. Alternativement, tous les composés de formule (I) comprennent une fonction chimique identique adaptée à greffer le composé correspondant sur un support adapté. En l'occurrence, l'un des groupes R¹, R², R³, R⁴, R⁵ ou R⁶ peut comporter une telle fonction, telle qu'un silane ou une amine, et les autres des groupes R¹, R², R³, R⁴, R⁵ o R⁶ peuvent être définis librement.

Selon un mode de réalisation alternatif, plusieurs composés de formules (I), liés au biosenseur, sont greffés sur plusieurs zones distinctes d'un même support. Dans ce cas, l'un des groupes R¹, R², R³, R⁴, R⁵ ou R⁶ destiné à réagir sur le support est identique pour l'ensemble des composés de formule (I) testés.

Il est entendu que, inversement, la fonction dicyanométhanide peut être dédiée au greffage des composé de formule (I) sur un support en polystyrène et que les groupes R¹, R², R³, R⁴, R⁵ o R⁶ peuvent être destinés à leur couplage avec des biomolécules ou des biosenseurs. Leur diversité permet ainsi d'optimiser l'adéquation du composé de formule (I), utilisé come lien, au biosenseur ou à la biomolécule.

Dans le cas où un composé de formule (I) est testé sur un polymère P tel que décrit plus haute, le greffage n'est pas nécessaire à l'élaboration du test diagnostique.

Il est entendu qu'un ou plusieurs des composés de formules (I) peuvent indépendamment être évalués pour le greffage d'un biosenseur ou d'une biomolécule sur un support solide ou pour la fonctionnalisation d'un polymère P tel que décrit ci-dessus, ou pour les deux.

L'étape d'évaluation peut comporter une étape de tests comparatifs a2"). Selon une mode de réalisation, les différents composés de formule (I) greffés au support et couplés au biosenseur, sont mis en contact direct ou indirect avec une biomolécule ou une molécule à tester. Des paramètres tels que la sensibilité, la fiabilité, la rapidité, la reproductibilité relatifs aux différents composé de formule (I) sont comparés. Les composés de formule (I) associés aux meilleurs résultats peuvent être identifiés et sélectionnés. Le suivi des réaction peuvent comprendre un test de colorimétrie ou une mesure optique dans le visible ou dans l'UV.

Selon un autre mode de réalisation, le polymère P forme avec un anticorps primaire A1 ou secondaire A2 un complexe de détection C d'un test de diagnostique, le complexe de détection étant lié directement ou indirectement à un biosenseur greffé sur un support solide. Les différents composés de formule (I), en solution, sont alors mis en contact avec ledit polymère P de sorte à réagir avec lui. Des paramètres tels que la sensibilité, la fiabilité, la rapidité, la reproductibilité relatifs aux différents composés de formule (I) sont comparés. Les composés de formule (I) associés aux meilleurs résultats peuvent être identifiés et sélectionnés. Le suivi des réaction peuvent comprendre un test de colorimétrie ou une mesure optique dans le visible ou dans l'UV.

L'élaboration d'un test de diagnostique peut comporter une étape b*"*) de sélection d'un ou plusieurs composés de formule (I) parmi les différents composés de formule (I) impliqués dans l'une ou plusieurs des étapes a"), a1") a2"). La sélection de tels composés peut prendre en considération l'un ou plusieurs des résultats des étapes a1") et a2"). Une pondération relative à l'une ou l'autre de ces étapes peut être considérée.

Sur la base d'un nombre restreint de composés de formule (I) déjà disponibles, étant utilisés comme liens de greffage sur une surface, ou comme produit révélateur en solution sur un complexe de détection, un test de diagnostique spécifique à la molécule à tester peut être élaboré rapidement et présenter une bonne sensibilité et/ou fiabilité. Il n'est alors pas nécessaire de produire un lien ou un révélateur totalement nouveau pour chaque diagnostique.

L'élaboration du test diagnostique peut en outre comporter une étape c*"*) de produire de nouveaux composés de formule (I) en fonction des indications recueillies lors des étapes précédentes, de sorte à optimiser ou améliorer les performances du test. Notamment, l'un ou plusieurs des groupes R¹, R², R³, R⁴, R⁵ o R⁶ peuvent être directement corrélés aux performance du composés de formule (I) pour une biomolécule donnée et optimisés en conséquence.

Selon un mode de réalisation, les résultats des tests sont stockés dans des bases de données de sorte à pouvoir faire l'objet d'analyses ultérieures. Selon un mode de réalisation, les bases de données peuvent être consultées par un programme d'intelligence artificielle et/ou de modélisation, de sorte à sélectionner et/ou produire des composés de formule (I) les plus adaptés à une biomolécule donnée.

Les modes de réalisations décrits ici n'ont pas vocation à être mutuellement exclusifs. Ils peuvent en l'occurrence se compléter, se chevaucher ou comporter des éléments communs. Ils peuvent être combinés totalement ou en partie dans la mesure des réalités pratiques.

### Exemples

### Oxyde de tetracyanoethylene (TCNEO)

Une solution de tetracyanoethylene (3.0 g, 34 mmol) dans 22 ml d'acétonitrile est refroidie à 5°C dans un bain d'acétone et de glace. 2.66 ml de peroxyde d'hydrogène (34 mmol) sont ajoutés goutte à goutte à une vitesse telle que la température du mélange reste comprise entre 10°C et 12°C. Le mélange est agité pendant encore 5 minutes après l'ajout du peroxyde d'hydrogène, puis dilué avec 150 ml d'eau glacée, de sorte à former un précipité solide blanc. Le précipité est séché sous vide et utilisé immédiatement.

### Carbonitrile-2-phthalazinium (la)

Une solution S1 comprenant 130.2 mg (2 mmol) de cyanure de potassium dissous dans 2 ml d'eau déminéralisée et une solution S2 de 2.5 ml de chlorure de benzoyl (2 mmol) dans 3 ml de dichlorométane sont préparées. Dans un ballon tricol muni d'une trappe à gaz, 65,7 mg (2 mmol) de phthalazine sont dissous dans 1.5 ml de dichlorométhane, puis la solution S2 est ajoutée goutte à goutte sous agitation. La solution S1 est ensuite ajoutée goutte à goutte et le mélange réactionnel est agité pendant 12 heures à température ambiante. 10 ml d'une solution saturée de bicarbonate de sodium sons ensuite ajoutés et le produit est extrait avec 6 ml de dichlorométhane. La phase organique est séché par du sulfate de sodium, filtrée et le solvant est évaporée.

### Phthalazinium-2-carboxylic acide (Ib)

650.7mg de phthalazine sont dissous dans 5ml de dichloromethane. Ensuite, 2.5 ml de chlorure de benzoyl sont mélangés à 3 ml de dichlorométhane. La solution est ajoutée goutte-à-goutte à celle de phthalazine sous agitation. Après, 1,3 g potassium cyanide sont dissous dans 2 ml d'eau deionisée et ajoutée goutte-à-goutte à la solution. La réaction est incubée à température ambiante et sous agitation pendant 15 heures. Ensuite, la réaction est transférée dans 20 ml d'une solution saturée en bicarbonate de et la solution organique extraite. La phase organique est séchée avec du sulfate de magnesium anhydre, filtrée, le solvant évaporé et le solide séché. Un solide blanc est obtenu.

500 mg du composé I sont solubilisés dans 5ml d'acide acétique. 3ml d'acide hydrobromique sont ajoutés goutte-à-goutte sous agitation. La réaction est laissée pendant 30 min sous agitation. Après l'incubation, la solution est neutralisés avec une solution d'hydroxyde de sodium 1M et extraite à l'acétate d'éthyle. La phase organique est séchée avec du sulfate de magnesium anhydre, filtrée et le solvant évaporé. Le solide obtenu est précipité 3x dans l'éther froid (5ml) et séché.

Le composé carbonitrile-2-phthalazinium (la) est hydrolysé par de l'acide bromhydrique dans l'acide acétique. Pour ce faire, 10 ml du carbonitrile-2-phthalazinium (la) sont solubilisés dans l'acide acétique et 1 ml d'acide bromhydrique est ajouté goutte à goutte sous agitation. La réaction est agitée pendant 30 minutes à température ambiante, neutralisée par 3 ml NaOH 1M, puis extraite avec 5 ml d'acétate d'éthyle. La phase organique est évaporée et le produit est purifié par trois recristallisations dans de l'éther froid.

### Phthalazinium-2-dicyanométhanide carboxylique acide 1,3-dipôle (I)

910 mg de Phthalazinium-2-carboxylic acide (Ib) sont dissous dans 40 ml d'acétate d'éthyle à une température inférieure à 0°C dans un bain de glace. La solution froide est traitée avec 5ml d'oxyde de tetracyanoethylene (TCNEO) (1.0 g, 7.0 mmol). Un précipité jaune se forme immédiatement et est collecté par filtration puis séché sous vide.

Spectroscopie FTIR: -COOH (3410 cm⁻¹)

RMN ¹H : Aromatiques 7,42-7,49(m, H1), 7,51-7-67 (m, H2, H3, H4), 8,16-8,19 (t, H5).

Spectrométrie de masse : M/z = 239

### Isobenzofuran-1(3H)-one

13g d'anhydride phtalique sont dissous dans 152 ml de tetrahydrofurane. La solution est refroidie à 0°C avant que 3.8g de NaBH₄ soit ajoutés par petite portion. La suspension blanche épaisse est mélangée pendant 12h à température ambiante. Après 12h, la solution est acidifiée par une solution de HCl 2M jusqu'à pH 1. La solution résultante est à nouveau incubée pendant 12h à température, et ensuite concentrée sous-vide pour obtenir un solide brun. Le produit brut est extrait avec de l'acetate d'éthyle (3x50 ml) et lavée avec une solution saturée de bicarbonate de sodium suivit par de la saumure (, 72%). Ensuite, la phase organique est séchée par sulfate de magnésium anhydre, filtrée et concentrée sous vide. Recristallisation dans l'éthanol donne le produit pur sous la forme de cristaux blancs.

### 3-Bromoisobenzofuran-1(3H)-one

2.6 g d'lsobenzofuran-1(3H)-one sont dissous dans 77 ml d'acetonitrile anhydre, ensuite 10.3 g de NBS et 9.1 g d'AIBN sont ajoutés. La réaction est mélangée pendant 12h à 75°C. Le précipité est est filtré et jeté tandis que le filtrat est concentré sous-vide. Le produit est purifié par colonne flash (1/0 → 3/1 éther de pétrole / acétate d'éthyle) et des cristaux incolores sont obtenus.

### Phthalazin-1(2H)-one

2 g de 3-bromoisobenzofuran-1(3H)-one sont dissous dans 10 ml de HCl 5 % (5 ml/mmol) et porté à reflux sous agitation pendant 2h. Ensuite, la réaction est refroidie à la température ambiante. Une fois la solution refroidie, 4 ml de solution d'hydrazine sont ajoutés (1.2 eq) et incubée pendant 2h sous agitation à température ambiante. Le précipité formé est récupéré et recristalliser dans l'éthanol chaud pour obtenir le produit pur.

### 2-chlorophthalazine

1g de phthalazine-1(2H)-one sont ajoutés à 10ml de POCl₃ et la solution est portée à 110°c sous agitation pendant 1h. Ensuite la solution est ajoutée goutte-à-goutte à de la glace pilée. Le précipité jaune qui s'est formé est filtré et lavé 3x avec de l'eau distillée puis finalement séché.

### Phthalazine-2-carboxylic acid

0.5g de 2-chlorophthalazine sont traités dans 5ml d'acide formique à 100°C pendant 4h sous agitation. Une fois la solution refroidie, le composé est extrait par HPLC préparative (gradient 0-70 %, eau/ACN, 10 min).

### Couplage avec le tryptophane

1 mg de tryptophane (4.9 µmole) est ajouté à 2 ml d'eau déminéralisée, puis 500 µl d'une solution de 1.3 mg du composé phthalazinium-2-dicyanométhanide carboxylique acide 1,3-dipôle (I) (4.9 µmol) dans de l'eau déminéralisée sont ajoutés. Le mélange réactionnel est chauffé à 80°C pendant 9 heures et caractérisé par LC/MS.

### Couplage avec le styrène

1 mg de styrène (9.6 µmole) est solubilisé dans 1.5 ml d'eau déminéralisé, puis 500 µl d'une solution de 2.3 mg du composé phthalazinium-2-dicyanométhanide carboxylique acide 1,3-dipôle (I) (9.6 µmole) dans de l'eau déminéralisée sont ajoutés. Le mélange réactionnel est chauffé à 80°C pendant 9 heures et caractérisé par LC/MS.

## Revendications

1. Composé de formule I 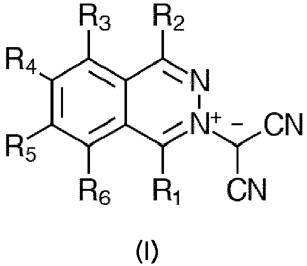 **Caractérisée en ce que** les substituants R¹, R², R³, R⁴, R⁵, et R⁶ désignent indépendamment l'un de l'autre un atome ou groupe d'atomes sélectionné parmi le groupe consistant en
- SO₃⁻, PO₄, NO₂, -NHS
- -(CH₂)ₓ-OR⁷, -(CH₂)ₓ-OH, -(CH₂)ₓ-NR⁷R⁸, -(CH₂)ₓ-NHR⁸, -(CH₂)ₓ-CONR⁷R⁸, - (CH₂)ₓ-CONHR⁸, -(CH₂)ₓ-CONH₂, -(CH₂)ₓ-CO₂NR⁷R⁸, -(CH₂)ₓ-CO₂NHR⁸, - (CH₂)ₓ-COR⁷, -(CH₂)ₓ-COH, -(CH₂)ₓ-COOR⁷, -(CH₂)ₓ-COOH, -(CH₂)ₓ-SR⁷, - (CH₂)ₓ-SH, -(CH₂)ₓ-SOR⁷, -(CH₂)ₓ-SOH, -(CH₂)ₓ-SO₂R⁷, -(CH₂)ₓ-SO₂H où x est un nombre entier compris entre 0 et 10,
- un atome d'halogène, et
- R⁷
où R⁷ désigne un atome ou groupe d'atomes sélectionné parmi le groupe consistant en
• un alkyl linéaire ou ramifié comprenant de 1 à 12 atomes de carbone, pouvant être saturé, mono-insaturé ou poly-insaturé, dans lequel 1 ou plusieurs atomes d'hydrogène peuvent être indépendamment l'un de l'autre remplacés par un atome d'halogène, un groupement -COR¹⁰, -CO₂R¹⁰, -OR¹⁰, -NO₂, NHR¹⁰, ou un groupement phényl éventuellement substitué par 1, 2, 3 ou 4 groupements indépendamment sélectionnés par le groupe consistant en -COR¹⁰, -CO₂R¹⁰, -OR¹⁰, -NO₂, -NHR¹⁰, N3,
• un cycloalkyl comprenant 4, 5, 6, 7, 8 ou 9 atomes de carbones, pouvant être saturé, mono-insaturé, doublement ou triplement insaturé, dans lequel 1 ou plusieurs atomes d'hydrogène peuvent être indépendamment l'un de l'autre remplacés par un atome d'halogène, un groupement -COR¹⁰, -CO₂R¹⁰, -OR¹⁰, - NO₂, NHR¹⁰, ou un groupement phényl éventuellement substitué par 1, 2, 3 ou 4 groupements indépendamment sélectionnés par le groupe consistant en -COR¹⁰, -CO₂R¹⁰, -OR¹⁰, -NO₂, NHR¹⁰ et
• un groupement aromatique comprenant 1 ou 2 cycles aromatiques éventuellement substitué par 1, 2, 3 ou 4 groupements indépendamment sélectionnés par le groupe consistant en -COR¹⁰, -CO₂R¹⁰, -OR¹⁰, -NO₂, NHR¹⁰, N₃, -CH(CN)₂,
où R⁸ désigne un atome ou groupe d'atomes sélectionné parmi le groupe consistant en
un atome d'hydrogène et
R₇,
ou bien dans laquelle R⁸ forme avec R⁷ dans les groupes -(CH₂)ₓ-NR⁷R⁸, -(CH₂)ₓ-CONR⁷R⁸, -(CH₂)ₓ-CO₂NR⁷R⁸, un hétérocycle comprenant de 4, 5, 6 ou 7 atomes de carbone, où 1 ou 2 groupes -CH₂- peuvent être indépendamment l'un de l'autre remplacés par -CO-, ou -CS-, et où deux atomes de carbone contigus peuvent être liés par une double liaison ;
où R¹⁰ désigne un atome d'hydrogène ou un groupe alkyl saturé comprenant de 1 à 4 atomes de carbone ;
où au moins l'un des substituants R¹, R², R³, R⁴, R⁵, et R⁶ est différent de H, ou bien si l'un des 6 substituants R¹, R², R³, R⁴, R⁵, et R⁶ est un atome de chlore, ou un groupe -OCH₃, -SCH₃, -CF₃, ou phenyl, alors au moins l'un des 5 autres substituants est différent de H, ou si 2 des substituants R¹, R², R³, R⁴, R⁵, et R⁶ sont des groupes -CH₃, alors au moins l'un des 4 autres substituants est différent de H.

2. Composé selon la revendication 1, **caractérisé en ce que**
- le substituant R⁶ désigne SO₃⁻, PO₄, NHS ou NO₂,
- les substituant R²et R⁵désignent indépendamment l'un de l'autre un atome ou un groupe d'atomes sélectionné dans le groupe consistant en un atome d'hydrogène, NHS, -(CH₂)ₓ-COOR⁷, -(CH₂)ₓ-COOH, -(CH₂)ₓ-CO₂NR⁷R⁸, où x est un nombre entier correspondant à 0 ou 1,
- les substituants R³ et R⁴ désignent indépendamment l'un de l'autre un atome ou un groupe d'atomes sélectionné dans le groupe consistant en un atome d'hydrogène, un atome d'halogène, -OR¹⁰ et -COR¹⁰,
où R⁷ désigne un atome ou groupe d'atomes sélectionné parmi le groupe consistant en un alkyl linéaire ou ramifié comprenant de 1 à 12 atomes de carbone, pouvant être saturé, mono-insaturé ou poly-insaturé, dans lequel 1 ou plusieurs atomes d'hydrogène peuvent être indépendamment l'un de l'autre remplacés par un atome d'halogène, un groupement-COR¹⁰, -CO₂R¹⁰, -OR¹⁰, -NO₂, -NHR¹⁰, ou un groupement phényl éventuellement substitué par 1, 2, 3 ou 4 groupements indépendamment sélectionnés par le groupe consistant en -COR¹⁰, -CO₂R¹⁰, -OR¹⁰, -NO₂, -NHR¹⁰,
ou bien dans laquelle R⁸ forme avec R⁷ dans le groupe -(CH₂)ₓ-CO₂NR⁷R⁸, un hétérocycle comprenant de 4, 5, ou 6 atomes de carbone, où 1 ou 2 groupes -CH₂- peuvent être indépendamment l'un de l'autre remplacés par -CO-, ou -CS-, et où deux atomes de carbone contigus peuvent être liés par une double liaison ;
où R¹⁰ désigne un atome d'hydrogène ou un groupe alkyl saturé comprenant de 1 à 4 atomes de carbone ;

3. Composé selon l'une ou l'autre des revendications 1 ou 2, **caractérisé en ce que**
- le substituant R¹ désigne un atome d'hydrogène,
- le substituant R⁶ désigne SO₃⁻ ou PO₄,
- les substituants R² et R⁵ désignent indépendamment l'un de l'autre un atome ou un groupe d'atomes sélectionné dans le groupe consistant en un atome d'hydrogène, NHS,-COOR⁷, -COOH, -CO₂NR⁷R⁸,
- les substituants R³ et R⁴ désignent indépendamment l'un de l'autre un atome ou un groupe d'atomes sélectionné dans le groupe consistant en un atome d'hydrogène, un atome de chlore, et un atome de fluor,
où R⁷ désigne un atome ou groupe d'atomes sélectionné parmi le groupe consistant en un alkyl saturé linéaire ou ramifié comprenant de 1 à 6 atomes de carbone, dans lequel 1 ou plusieurs atomes d'hydrogène peuvent être indépendamment l'un de l'autre remplacés par un atome d'halogène, un groupement -COR¹⁰, -CO₂R¹⁰, -OR¹⁰, -NO₂, -NHR¹⁰,
ou bien dans laquelle R⁸ forme avec R⁷ dans le groupe -CO₂NR⁷R⁸, un hétérocycle comprenant de 4, ou 5 atomes de carbone, où 1 ou 2 groupes - CH₂- peuvent être indépendamment l'un de l'autre remplacés par -CO-, ou - CS-, et où deux atomes de carbone contigus peuvent être liés par une double liaison ;

4. Composé selon la revendication 1, **caractérisé en ce que** le composé est sélectionné parmi l'un des composés suivants :
| | | | |
|---|---|---|---|
| P1 | 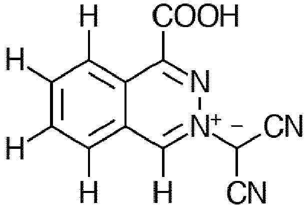 | P2 | 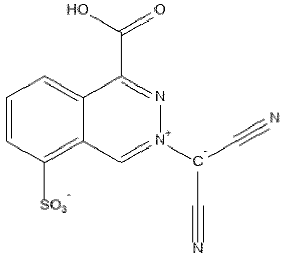 |
| P3 | 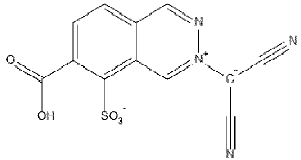 | P4 | 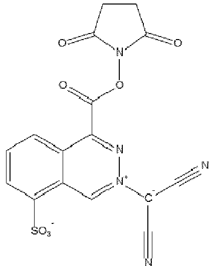 |
| P5 | 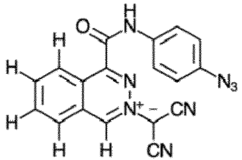 | P6 | 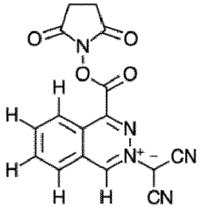 |
| P7 | 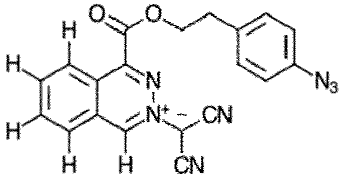 | P8 | 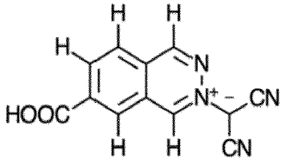 |
| P9 | 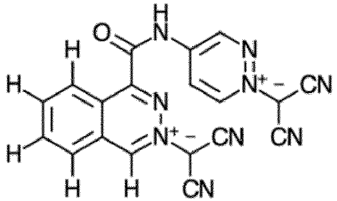 | P10 | 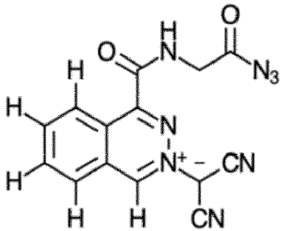 |
| P11 | 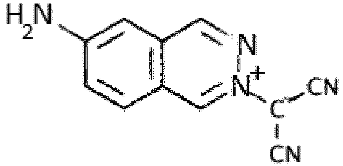 | P12 | 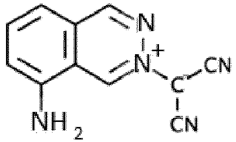 |

5. Kit de test ou de diagnostique comprenant au moins un composé de formule (I) tel que décrit dans l'une des revendications 1 à 4, au moins un support solide et au moins un biosenseur.

6. Kit de test ou de diagnostique selon la revendication 5, ledit au moins un support comprenant au moins une fonction chimique propre à réagir avec l'un ou l'autre des groupes R¹, R², R³, R⁴, R⁵, R⁶ ou dicyanométhanide du ou des composés de formule (I), et ledit au moins un biosenseur comprenant une ou plusieurs fonctions chimiques propres à réagir avec un autre des groupes R¹, R², R³, R⁴, R⁵, R⁶ ou dicyanométhanide, de sorte que ledit composé de formule (I) soit adapté à greffer ledit biosenseur sur ledit au moins un support.

7. Kit de test ou de diagnostique selon les revendications 5 ou 6, comprenant en outre au moins un complexe de détection (C) formé d'un anticorps primaire (A1) ou secondaire (A2) fonctionnalisé par un polymère (P) comportant au moins une fonction chimique adaptée à réagir avec l'un ou l'autre des groupes R¹, R², R³, R⁴, R⁵, R⁶ ou dicyanométhanide d'un composé de formule (I), ledit complexe étant adapté à se lier directement ou indirectement avec un épitope (E) du au moins un biosenseur, l'un au moins desdits composés de formule (I) pouvant être mis en solution de sorte à réagir avec la au moins une fonction chimique du polymère (P).

8. Kit selon l'une des revendications 5 à 7, où ledit au moins un composé de formule (I) étant libre en solution, confère à ladite solution une coloration dépendante ou proportionnelle de la concentration dudit au moins un composé de formule (I).

9. Kit selon l'une des revendications 5 à 8, ledit au moins un support comportant plusieurs zones distinctes de greffage.

10. Procédé pour la production du composé de formule (I) tel que décrit dans l'une des revendications 1 à 4, 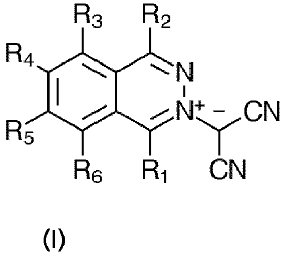
où les substituants R¹, R², R³, R⁴, R⁵, et R⁶sont tels que décrits dans l'une des revendications 1 à 4,
**caractérisé en ce que** le composé de formule Ib, où les substituants R¹, R², R³, R⁴, R⁵, et R⁶ sont tels que décrits dans l'une des revendications 1 à 4, 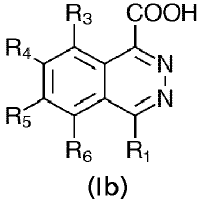
est mis en réaction avec de l'oxyde de tetracyanoéthylène.

11. Procédé pour la production de peptides ou protéines fonctionnalisées au moyen d'un produit de formule (I), selon l'une des revendications 1 à 4, **caractérisé en ce que** le produit de formule (I) est mis en réaction avec l'insaturation d'un ou plusieurs reste Tryptophane dudit peptide ou de ladite protéine, de sorte que la fonction dicyanométhanide dudit composé de formule (I) réagisse spontanément avec l'insaturation du tryptophane, de manière à obtenir un produit de formule (III), dont les substituants R¹, R², R³, R⁴, R⁵, et R⁶ sont tels que décrits dans l'une des revendications 1 à 4.

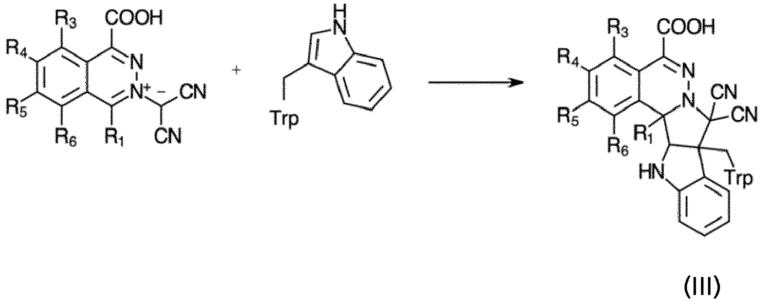

12. Procédé selon la revendication 11, dans lequel l'un ou plusieurs des groupes R¹, R², R³, R⁴, R⁵ et R⁶ sont fonctionnalisés de sorte à réagir avec un support solide conduisant à greffer ledit peptide ou protéine sur ledit support, ou avec une autre molécule conduisant à coupler ledit peptide ou protéine avec ladite autre molécule.

13. Procédé pour le greffage d'une molécule de formule (I) selon l'une des revendications 1 à 4 sur un support solide comprenant du styrène, **caractérisé en ce que** le produit de formule (I) est mis en réaction avec le support solide de sorte que sa fonction dicyanométhanide réagisse spontanément avec l'insaturation du styrène du support solide, pour former un produit de formule (IV) dans lequel les groupes R¹, R², R³, R⁴, R⁵ et R⁶ sont tels que définis dans l'une des revendications 1 à 4.

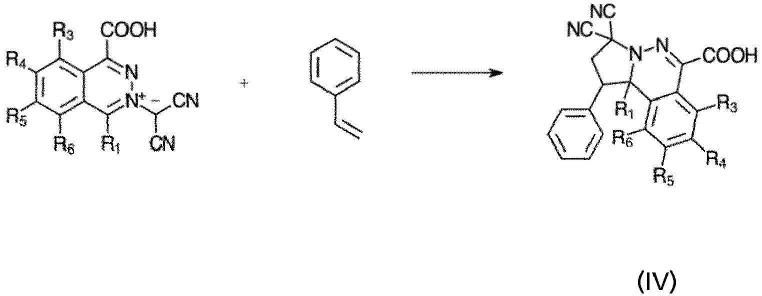

14. Procédé selon la revendication 13, dans lequel l'un ou plusieurs des groupes R¹, R², R³, R⁴, R⁵ et R⁶ sont fonctionnalisés de sorte à réagir avec une biomolécule, un biosenseur ou une molécule à tester, conduisant ainsi au greffage de ladite biomolécule, biosenseur ou molécule à tester sur ledit support.

15. Procédé de fonctionnalisation d'un polymère (P) comprenant au moins une fonction chimique adaptée à réagir avec au moins l'un des groupes R¹, R², R³, R⁴, R⁵, R⁶ ou dicyanométhanide d'un composé de formule (I), ledit polymère (P) formant avec un anticorps primaire (A1) ou secondaire (A2) un complexe de détection (C), ledit procédé comprenant une étape de faire réagir ledit composé de formule (I) en solution sur ledit polymère (P).

16. Procédé d'élaboration d'un test diagnostique sur support solide, comprenant l'évaluation d'un ou plusieurs composés de formule (I) parmi une librairie de composés dans le greffage d'un biosenseur ou d'une biomolécule sur un support solide selon l'une des revendications 13 et 14, ou dans la fonctionnalisation d'un polymère (P) selon la revendication 15, les résultats desdites étapes de greffage et/ou de fonctionnalisation étant comparés de sorte à sélectionner le ou les meilleurs composés de formule (I) pour une biomolécule donnée.

17. Procédé selon l'une des revendications 11 à 16, ledit au moins un composé de formule (I) étant libre en solution, confère à ladite solution une coloration dépendante ou proportionnelle de sa concentration, ledit procédé incluant au moins une étape de détermination de la concentration du au moins un composé de formule (I) dans la solution via une analyse colorimétrique ou optique.
